# FASCICULE DE BREVET EUROPEEN

(11) **EP 2 768 398 B1**
(45) Date de publication et mention de la délivrance du brevet: **13.06.2018**
(21) Numéro de dépôt: 12772975.4
(22) Date de dépôt: 18.10.2012
(51) Int. Cl.: A61B 17/06, A61B 10/02, A61B 17/34, A61B 17/00

(54) **DISPOSITIF DE GUIDAGE D'UNE AIGUILLE MEDICALE**
VORRICHTUNG ZUR FÜHRUNG EINER MEDIZINISCHEN KANÜLE
DEVICE FOR GUIDING A MEDICAL NEEDLE

(30) Priorité: 18.10.2011 FR 1159387
(43) Date de publication de la demande: 27.08.2014
(73) Titulaire: Université Grenoble Alpes, 38400 Saint-Martin-d'Hères (FR)
(72) Inventeur: FAVIER, Denis, F-38410 Saint Martin d'Uriage (FR); ALONSO, Thierry, F-38330 Saint Ismier (FR); CHAGNON, Grégory, F-73110 Detrier (FR); LIU, Yinong, Nedlands, Western Australia 6009 (AU); MOREAU-GAUDRY, Alexandre, F-38240 Meylan (FR)
(74) Mandataire: Regimbeau
(86) Numéro de dépôt international: PCT/EP2012/070651
(87) Numéro de publication internationale: WO 2013/057189

(56) Documents cités:
- WO-A1-2006/065913
- WO-A1-2007/056590
- WO-A2-2007/059233
- US-A- 5 231 989
- US-A1- 2004 133 168
- US-A1- 2011 087 257

## Description

### DOMAINE DE L'INVENTION

La présente invention concerne un dispositif de guidage d'une aiguille médicale et un procédé de fabrication d'un tel dispositif.

### ARRIERE PLAN DE L'INVENTION

Les aiguilles médicales sont fréquemment utilisées lors d'interventions chirurgicales sur un organisme vivant, en vue, par exemple, de réaliser des ponctions, des injections, des biopsies, etc. dans une cible située à l'intérieur de l'organisme.

Une aiguille médicale est un corps creux tubulaire allongé.

Généralement, l'aiguille présente une extrémité biseautée pour faciliter le passage à travers les tissus.

Selon l'intervention, la cible peut être située à une profondeur de 2 à 40 cm sous la peau du patient.

Cependant, la trajectoire que peut emprunter l'aiguille jusqu'à la cible située à l'intérieur de l'organisme n'est pas nécessairement linéaire.

Il est en effet possible que des obstacles soient interposés entre le point d'insertion de l'aiguille et la cible.

De tels obstacles peuvent être des os (que l'aiguille ne peut traverser), des organes sensibles (qui risqueraient d'être endommagés par l'aiguille), etc.

Par ailleurs, il a été démontré que, lors de son insertion, l'aiguille était sujette à des déformations.

Cette courbure de l'extrémité biseautée affecte donc la trajectoire de l'aiguille ; la trajectoire réelle étant différente de la trajectoire prévue par le praticien.

On pourra à cet égard se référer aux travaux de Webster et al., "Nonhlonmic modeling of needle steering", International Journal of Robotics Research, Sage Publications Ltd, 2006, 25, pp. 509-525.

Le praticien cherche donc à pouvoir guider l'aiguille selon une trajectoire maîtrisée en fonction des obstacles qui s'interposent entre le point d'insertion et la cible.

Des dispositifs de guidage visant à déformer l'aiguille pour contourner les obstacles prévus ont donc été développés.

Ainsi, le document WO 2010/020591 divulgue une aiguille médicale équipée d'actionneurs fixés sur la surface externe de l'aiguille ou incorporés à sa paroi par des techniques de micro-fabrication.

Lesdits actionneurs permettent d'imposer une contrainte locale à l'aiguille en vue d'agir sur la courbure de son extrémité distale.

Cependant, la fabrication de ces actionneurs miniatures reste délicate et onéreuse.

Le document US 2009/0270676 divulgue un dispositif de guidage d'une aiguille, comprenant une canule externe dont une portion distale est susceptible d'être courbée par un système d'actionnement à câble. L'aiguille, qui présente une rigidité inférieure à celle de la canule externe, se conforme à la courbure de la canule externe au fur et à mesure qu'elle est avancée dans celle-ci et reprend une forme droite une fois que son extrémité distale dépasse de celle de la canule externe.

L'article de T.R. Wedlick et al., "Characterization of Pre-Curved Needles for Steering in Tissue", 31st Annual International Conférence of the IEEE EMBS Minneapolis, 26 Sept. 2009 présente des travaux visant à déterminer l'influence du rayon de courbure et de l'angle de courbure sur la trajectoire d'une aiguille dont l'extrémité distale est pré-courbée.

Cependant, l'extrémité de ladite aiguille est courbée de manière permanente, ce qui limite les possibilités de modifier la trajectoire en fonction des obstacles à éviter.

L'article "A steerable Needle Technology Using Curved Concentric Tubes", P. Sears et al., Proceedings of the 2006 IEEE/RSJ International Conférence on Intelligent Robots and Systems, Oct. 9-15, 2006, pp. 2850-2856 décrit une aiguille médicale télescopique constituée d'une pluralité de tubes concentriques courbes, ce qui permet de conférer à l'aiguille un trajet tridimensionnel à l'intérieur du corps du patient pour éviter des os ou des organes sensibles.

Selon la rigidité respective des différents tubes, la courbure de l'aiguille peut être imposée par la courbure du tube le plus rigide ou par la combinaison des courbures de tubes de rigidité équivalente.

Cependant, un tel agencement de tubes modifie la courbure générale de l'aiguille et n'agit pas spécifiquement sur la courbure de l'extrémité distale.

Par ailleurs, cette construction a pour effet, pour un diamètre intérieur donné, d'augmenter le diamètre extérieur de l'aiguille.

Or, on cherche au contraire à minimiser le diamètre extérieur de l'aiguille afin de limiter les traumatismes imposés à l'organisme et à faciliter les suites postopératoires.

Le document US 2004/0133168 divulgue un dispositif de guidage d'une aiguille selon le préambule de la revendication 1, ainsi qu'un procédé de fabrication selon le préambule de la revendication 8. Il subsiste donc un besoin de concevoir un dispositif de guidage d'une aiguille médicale qui ne conduise pas à augmenter le diamètre de l'aiguille, qui soit aisé et peu onéreux à fabriquer et qui permette d'imposer à l'aiguille et/ou à l'insert au moins une courbure localisée.

### BREVE DESCRIPTION DE L'INVENTION

Conformément à l'invention, il est proposé un dispositif de guidage d'une aiguille médicale, comprenant les caractéristiques de la revendication 1. Par « comportement mécanique » on entend dans le présent texte la relation entre la contrainte et la déformation d'un matériau, à une température donnée.

Dans l'application visée, la température considérée est avantageusement la température du corps humain ou animal dans lequel l'aiguille est destinée à être guidée.

Par « déformation localisée » on entend une variation de la courbure d'une partie seulement de l'aiguille et/ou de l'insert.

Comme cela sera exposé plus bas, l'une au moins de ces régions est réalisée par un traitement thermique ou thermomécanique localisé de l'aiguille.

Selon un mode de réalisation, l'aiguille présente aux moins deux régions de rigidités différentes, l'une au moins desdites régions présentant une rigidité supérieure à celle de la portion courbée de l'insert.

Selon une forme d'exécution ne faisant pas partie de l'invention, lesdites régions forment des portions de l'aiguille adjacentes le long d'un axe longitudinal de l'aiguille.

Selon l'invention, lesdites régions sont adjacentes sur la circonférence de la portion distale de l'aiguille.

De manière avantageuse, l'insert présente une forme cylindrique pleine de section circulaire. Selon l'invention, l'aiguille et l'insert sont en des métaux biocompatibles élastiques, élastoplastiques ou super-élastiques à la température du corps humain.

Selon un mode de réalisation préféré de l'invention, l'aiguille et/ou l'insert sont dans un alliage nickel-titane.

Selon une forme d'exécution particulière de l'invention, l'aiguille et l'insert présentent une épaisseur constante.

Le diamètre extérieur de l'aiguille peut être compris entre 0,3 et 2 mm et le diamètre de l'insert peut quant à lui être compris entre 0,1 et 1,8 mm.

Selon un mode de réalisation de l'invention, l'aiguille présente, de son extrémité distale vers son extrémité proximale, une première portion dont la rigidité est supérieure à la rigidité de la portion courbée de l'insert et une deuxième portion dont la rigidité est inférieure à la rigidité de la portion courbée de l'insert.

Un autre objet concerne un procédé de fabrication d'un dispositif de guidage d'une aiguille médicale, le procédé comprenant les caractéristiques de la revendication 8. De manière particulièrement avantageuse, le traitement thermique et/ou thermomécanique appliqué à l'aiguille forme au moins deux régions de rigidités différentes, l'une au moins desdites portions présentant une rigidité supérieure à la rigidité de la portion courbée de l'insert.

De manière préférée, ledit traitement thermique ou thermomécanique est réalisé dans une atmosphère neutre.

Selon une forme d'exécution particulière, ledit traitement thermique ou thermomécanique comprend une irradiation localisée de l'aiguille par un laser.

### BREVE DESCRIPTION DES DESSINS

D'autres caractéristiques et avantages de l'invention ressortiront de la description détaillée qui va suivre, en référence aux dessins annexés sur lesquels :
- la figure 1 est une vue en coupe du dispositif de guidage de l'aiguille selon un mode de réalisation ne faisant pas partie de l'invention ;
- les figures 2A et 2B sont des vues en coupe du dispositif de la figure 1 selon différentes positions relatives de l'aiguille et de l'insert ;
- la figure 3 présente des courbes de la contrainte en fonction de la déformation qui pourront être retenues pour les différentes zones de l'insert et l'aiguille ;
- la figure 4 est une vue en coupe d'un deuxième mode de réalisation ne faisant pas partie de l'invention ;
- les figures 5A et 5B sont des vues en coupe d'un troisième mode de réalisation ne faisant pas partie de l'invention, selon différentes positions relatives de l'aiguille et de l'insert ;
- les figures 6A à 6C sont des vues de profil, de face et de côté d'une aiguille selon un mode de réalisation de l'invention ;
- la figure 7 est une vue de profil d'un insert de guidage de l'aiguille illustrée aux figures 6A à 6C ;
- la figure 8 illustre les positions angulaires respectives de l'aiguille et de l'insert ;
- les figures 9A et 9B illustrent deux courbures différentes susceptibles d'être obtenues avec l'aiguille des figures 6A à 6C et l'insert de la figure 7 ;
- la figure 10 illustre de manière schématique la mise en oeuvre d'un traitement thermique localisé de l'aiguille ;
- la figure 11 illustre de manière schématique la mise en oeuvre d'un traitement thermomécanique de l'aiguille.

### DESCRIPTION DETAILLEE DE L'INVENTION

### Dispositif de guidage

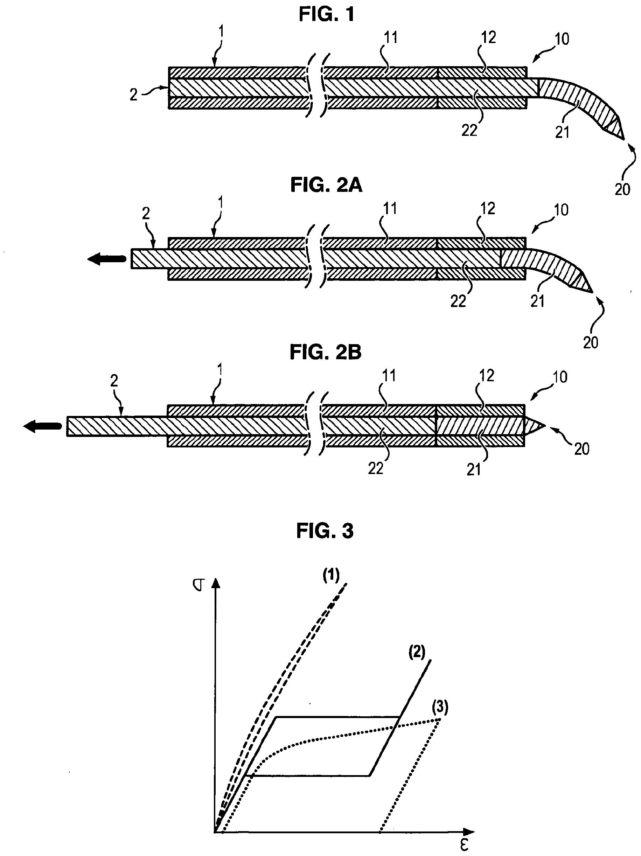

En référence à la figure 1, le dispositif de guidage de l'aiguille médicale comprend l'aiguille 1 elle-même et un insert 2 adapté pour coulisser en translation et en rotation à l'intérieur de l'aiguille 1.

L'insert présente une forme cylindrique pleine de section circulaire, dont le diamètre est légèrement inférieur au diamètre intérieur de l'aiguille 1, de manière à pouvoir coulisser sans frottement à l'intérieur de l'aiguille 1.

L'aiguille 1 présente une forme tubulaire de section circulaire, dont l'extrémité distale 10 (i.e. l'extrémité destinée à traverser les tissus jusqu'à la cible) peut être biseautée, bien que le biseau ne soit pas visible sur la figure 1.

L'aiguille peut présenter toutes dimensions (longueur, diamètre intérieur et extérieur) adaptées en fonction de l'usage auquel elle est destinée.

Par exemple, et de manière non limitative, le diamètre intérieur de l'aiguille peut typiquement être compris entre 0,1 et 1,6 mm, son diamètre extérieur peut être compris entre 1 et 1,8 mm, et sa longueur peut être comprise entre 3 et 40 cm.

L'aiguille 1 et l'insert 2 sont réalisés dans des matériaux qui sont adaptés pour changer de comportement sous l'effet d'un traitement thermique ou thermomécanique.

Ceci permet de fabriquer l'aiguille et l'insert chacun dans un même matériau sur toute leur longueur, puis de modifier localement leur comportement mécanique - et, dans le cas de l'insert, leur courbure - au moyen d'un traitement thermique ou thermomécanique.

Les alliages nickel-titane sont connus pour leurs propriétés de mémoire de forme et sont donc particulièrement préférés pour la mise en oeuvre de l'invention.

Notamment, ces alliages possèdent, à la température du corps humain, des propriétés super-élastiques qui leur permettent de se déformer avec une grande amplitude (de l'ordre de 10%) de manière non permanente.

Par ailleurs, ils ont l'avantage de pouvoir présenter des propriétés très différentes selon les traitements thermiques qu'ils ont subis, ce qui offre une vaste gamme de comportements illustrés schématiquement sur la figure 3 qui sera décrite en détail plus bas.

Cependant, selon les applications du dispositif, on pourra choisir d'autres matériaux appropriés dans la gamme des aciers chirurgicaux pour l'insert et/ou l'aiguille, par exemple de l'acier 316L.

Comme on peut le voir sur la figure 1, l'insert 2 présente, à l'état libre (c'est-à-dire hors de l'aiguille) une portion 21 adjacente à son extrémité distale 20 qui est courbée.

Ladite courbure est obtenue par l'application d'un traitement thermomécanique localisé à l'extrémité distale de l'insert.

Le reste 22 de l'insert est rectiligne à l'état libre.

L'extrémité distale 20 de l'insert présente de préférence un embout de forme pyramidale, conique voire biseautée.

Cet embout permet de boucher le diamètre intérieur de l'aiguille et ainsi d'éviter un phénomène de carottage au cours de l'insertion de l'aiguille à travers les tissus.

Par ailleurs, l'aiguille 1 présente au moins deux régions 11, 12 présentant des comportements mécaniques différents.

Lesdites régions peuvent être des portions de l'aiguille, c'est-à-dire qu'elles s'étendent sur toute la circonférence de l'aiguille sur une partie déterminée de la longueur de l'aiguille. Ces régions sont alors adjacentes le long d'un axe longitudinal de l'aiguille.

De manière alternative, lesdites régions peuvent appartenir à une même portion de l'aiguille en s'étendant chacune sur une partie de la circonférence de l'aiguille.

Le comportement mécanique des différentes régions 11, 12 de l'aiguille et des portions 21, 22 de l'insert est choisi de sorte qu'en fonction de la position de l'insert 2 à l'intérieur de l'aiguille 1, on peut imposer à l'aiguille 1 différentes déformations de sa portion distale et donc différentes trajectoires.

Dans le mode de réalisation illustré à la figure 1, la région 11 correspond à la majeure partie de la longueur de l'aiguille tandis que la région 12 est la partie de l'aiguille adjacente à l'extrémité distale 10.

De manière avantageuse, la longueur de la région 12 est de l'ordre de la longueur de la portion courbée 21 de l'insert 2.

Les figures 2A et 2B présentent deux positions relatives de l'aiguille et de l'insert du dispositif de la figure 1.

Comme illustré aux figures 1 et 2A, lorsque la portion courbée 21 de l'insert 2 dépasse (totalement ou en partie) de l'extrémité distale 10 de l'aiguille, l'aiguille 1 est rectiligne.

Lors de l'insertion de l'aiguille, c'est donc la portion courbée 21 de l'insert 2 qui impose principalement la trajectoire de l'aiguille.

Comme illustré à la figure 2A, lorsque la portion courbée 21 est partiellement rentrée dans l'aiguille, l'aiguille est pratiquement rectiligne, c'est-à-dire qu'elle est, dans sa région 12, plus rigide que la portion courbée 21 de l'insert.

La partie courbée de l'insert dépassant de l'extrémité distale 10, qui est la partie effective pour le guidage, présente alors une courbure moins prononcée que lorsque la totalité de la portion courbée 21 de l'insert dépasse de l'extrémité distale 10 de l'aiguille.

Le praticien peut donc imposer à l'aiguille une trajectoire présentant une courbure plus ou moins prononcée en sortant plus ou moins la portion courbée 21 de l'insert par rapport à l'extrémité distale 10 de l'aiguille.

Comme illustré à la figure 2B, lorsque l'extrémité distale 20 de l'insert 2 est située au niveau de l'extrémité distale 10 de l'aiguille 1, la portion courbée 21 de l'insert est entourée par la région 12 de l'aiguille 1.

La région 12 de l'aiguille présentant une rigidité supérieure à celle de la portion courbée 21 de l'insert, elle reste pratiquement rectiligne.

La trajectoire de l'aiguille dans cette configuration est donc rectiligne.

Un choix judicieux des comportements mécaniques de la région 12 de l'aiguille et de la portion courbée 21 de l'insert, qui sont obtenus grâce à des traitements thermiques ou thermomécaniques localisés de l'aiguille et de l'insert, permet donc d'obtenir la courbure souhaitée en fonction de la position axiale de l'insert 2 par rapport à l'aiguille 1.

La figure 3 présente les courbes de comportement (contrainte σ (en MPa) en fonction de la déformation ε (en %)) que l'on peut retenir pour les différentes portions de l'insert et de l'aiguille.

De manière bien connue, ces courbes sont tracées au moyen d'essais de traction sur une éprouvette normalisée réalisée dans le matériau considéré.

La courbe (1) correspond à un comportement élastique pouvant être obtenu, par exemple, à partir d'un fil ou d'un tube de nickel-titane brut d'étirage.

La courbe (2) correspond à un comportement superélastique pouvant être obtenu, par exemple, à partir d'un fil ou d'un tube de nickel-titane brut d'étirage, auquel on a appliqué un traitement thermomécanique.

La courbe (3) correspond à un comportement élastoplastique classique pour de nombreux matériaux.

La pente de ces courbes et la valeur des « plateaux » que l'on peut observer pour les courbes (2) et (3) peuvent être modulés par l'application de traitements thermiques ou thermomécaniques du matériau.

Il est ainsi possible de conférer aux portions 12 et 21 de l'aiguille et de l'insert, des comportements locaux différenciés, de telle sorte qu'un déplacement de l'insert à l'intérieur de l'aiguille permette de modifier la déformation et donc la trajectoire de ladite aiguille.

Ainsi, le comportement de la portion courbée 21 de l'insert est du type (1) ou (2) dans la gamme des lois de comportements illustrées à la figure 3.

Le comportement de la région 12 de l'aiguille est l'un des comportements de type (1), (2) ou (3) illustrés à la figure 3.

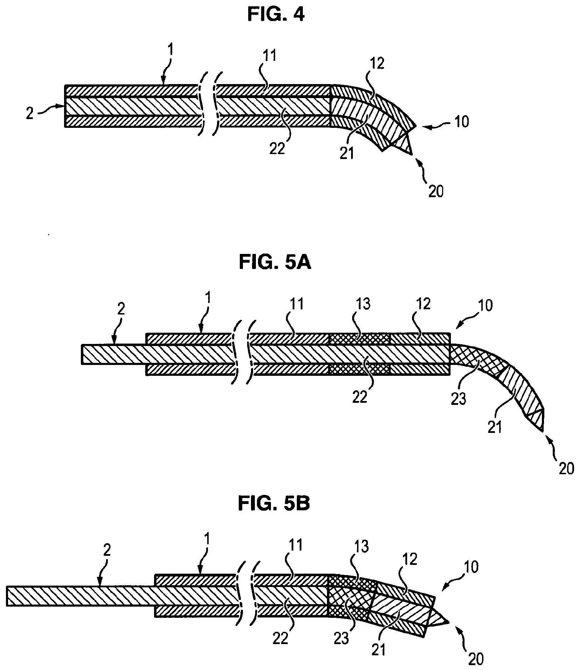

La figure 4 illustre une autre configuration possible de l'aiguille et de l'insert.

Dans ce cas, l'aiguille présente une région distale 12 qui présente une rigidité inférieure à celle du reste 11 de l'aiguille.

L'insert présente quant à lui une portion distale courbée 21.

Dans cet exemple, l'aiguille est réalisée en acier 316L, qui est un matériau fréquemment employé à l'heure actuelle pour les aiguilles médicales.

Cependant, contrairement aux aiguilles conventionnelles, la région 12 a subi un recuit localisé tandis que le reste 11 de l'aiguille n'en a subi aucun.

Ce recuit a pour effet de diminuer localement la rigidité de l'aiguille.

Par rapport à un alliage nickel-titane, l'acier 316L présente l'avantage d'être moins onéreux ; en revanche, il accepte des déformations élastiques beaucoup plus limitées (environ dix fois moindres que celles acceptées par un alliage nickel-titane).

L'insert est réalisé en un alliage nickel-titane similaire à celui décrit dans l'exemple précédent.

La région distale 12 de l'aiguille présente une rigidité inférieure à celle de la portion courbée 21 de l'insert, de sorte qu'elle est courbée lorsqu'elle entoure ladite portion 21 de l'insert.

Naturellement, on pourra définir d'autres configurations des différentes portions de l'aiguille et de l'insert, et d'autres comportements élastiques, que ceux illustrés aux figures 1 à 2B et 4.

Ainsi, par exemple, les positions respectives des portions plus rigides et moins rigides de l'aiguille et de l'insert peuvent être différentes de celles de l'exemple illustré à la figure 1, selon les applications visées.

Par ailleurs, l'aiguille et l'insert pourront présenter plus de deux régions présentant des comportements différents.

Un tel mode de réalisation, non limitatif, est illustré aux figures 5A et 5B.

Dans cet exemple, l'aiguille 1 présente trois régions 11, 12, 13 présentant des comportements mécaniques différents.

Par rapport à l'aiguille illustrée à la figure 1, l'aiguille présente une troisième région 13 située entre la région 11 et la région distale 12.

Grâce à un traitement thermique ou thermomécanique approprié, la région 13 présente une rigidité inférieure à celle de la portion 12.

L'insert 2 présente quant à lui trois portions 21, 22, 23 présentant des comportements mécaniques différents.

Par rapport à l'insert illustré à la figure 1, l'insert présente une troisième portion 23 située entre la portion 22 et la portion distale 21.

Grâce à un traitement thermique ou thermomécanique approprié, la portion 23 présente une rigidité supérieure à celle de la portion 21.

Comme on peut le voir à la figure 5A, les portions 23 et 21 sont courbées à l'état libre (c'est-à-dire lorsqu'elles dépassent de l'extrémité distale 10 de l'aiguille 1).

Par ailleurs, les régions 13 et 12 de l'aiguille présentent respectivement une longueur sensiblement égale à la longueur des portions 23 et 21.

Lorsque l'on rentre l'insert 2 à l'intérieur de l'aiguille 1 (comme illustré à la figure 5B), les portions 23 et 21 de l'insert sont respectivement entourées par les régions 13 et 12 de l'aiguille.

Grâce aux comportements différents de ces différentes portions, la région 13 de l'aiguille se courbe (dans une moindre mesure que la portion 23 à l'état libre), tandis que la région 12 reste rectiligne.

Le praticien peut ainsi courber localement l'aiguille à une certaine distance de son extrémité distale, tout en conservant rectiligne une portion distale de l'aiguille.

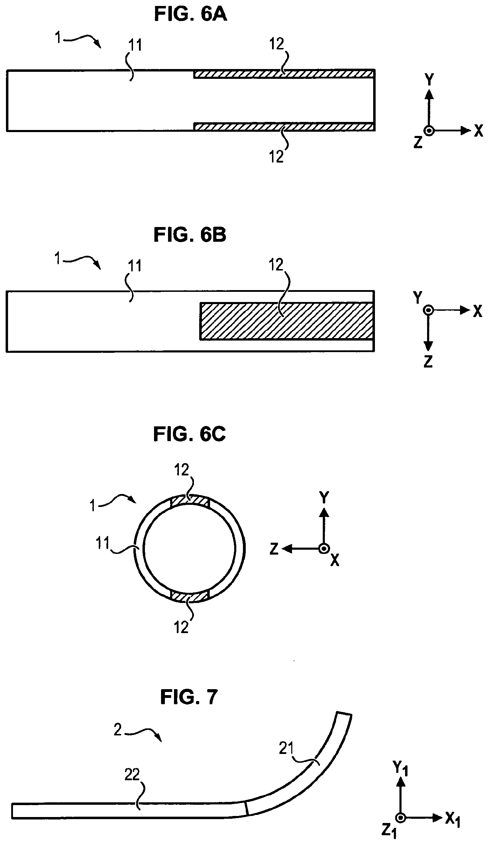

Les figures 6A à 6C illustrent un autre mode de réalisation de l'aiguille, l'aiguille étant représentée respectivement en vue de profil, en vue de dessus et en vue de côté, dans un repère orthogonal x, y, z.

Dans ce mode de réalisation selon l'invention, deux régions 12 présentant un comportement mécanique différent de celui du reste 11 de l'aiguille s'étendent le long de deux génératrices diamétralement opposées de l'aiguille.

Chacune desdites régions 12 ne s'étend que sur une partie de la circonférence de l'aiguille ; les deux régions 12 sont donc séparées par deux régions 11 qui présentent le même comportement mécanique que le reste de l'aiguille.

Par exemple, les régions 12 sont formées par un traitement thermique qui a pour effet de diminuer localement la rigidité de l'aiguille.

L'insert 2 est quant à lui représenté à l'état libre sur la figure 7, dans un repère orthogonal x1, y1, z1, la portion rectiligne 22 de l'insert s'étendant dans la direction de l'axe x1 et la portion courbée 21 de l'insert s'étendant dans le plan (x1, y1).

La courbure de l'insert peut donc être définie par les coordonnées des extrémités de la portion courbée 21 dans le plan (x1, y1).

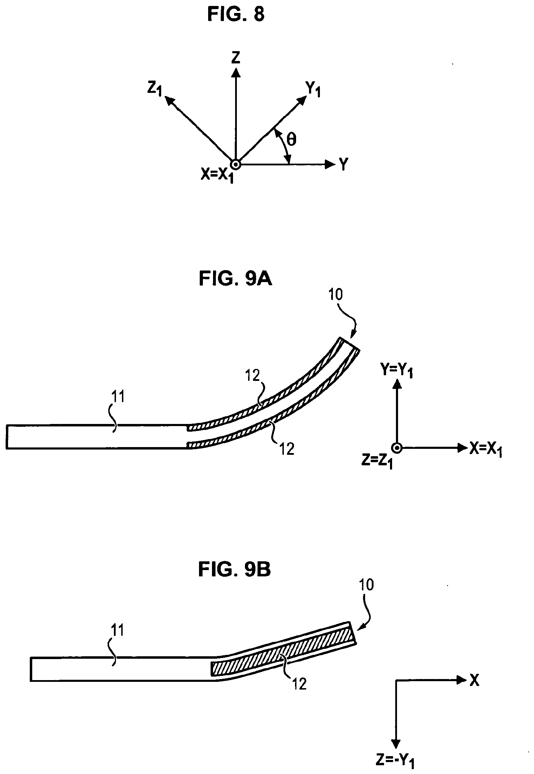

Lorsque l'on introduit l'insert dans l'aiguille, les repères (x, y, z) de l'aiguille et (x1, y1, z1) de l'insert possèdent un axe longitudinal commun (x = x1), et les deux autres axes sont susceptibles d'être décalés d'un angle θ, comme cela est illustré à la figure 8.

Le comportement mécanique de l'aiguille n'étant pas homogène sur toute la circonférence de sa portion distale, on peut obtenir une courbure différente de l'aiguille en faisant varier la position angulaire θ de la portion courbée 21 de l'insert par rapport à l'aiguille.

Ainsi, comme illustré à la figure 9A, si l'on aligne les axes y de l'aiguille et y1 de l'insert (soit θ = 0°), on obtient une courbure de l'aiguille relativement prononcée, quoique présentant un rayon de courbure inférieur à celui de la portion courbée de l'insert à l'état libre.

En effet, la courbure de la portion courbée 21 de l'insert est dans le même plan que les régions 12 moins rigides de l'aiguille, ce qui favorise une déformation localisée de l'aiguille.

En revanche, si, comme illustré à la figure 9B, on rend perpendiculaire les axes y de l'aiguille et y1 de l'insert (soit θ = -90°), on obtient une courbure moins prononcée de l'aiguille que dans le cas de la figure 9A.

En effet, la courbure de la portion courbée 21 de l'insert s'étend dans le même plan que les régions 11 qui présentent le même comportement mécanique que le reste de l'aiguille, et qui sont plus rigides que les régions 12.

On précise que les figures 9A et 9B représentent la situation dans laquelle l'insert est totalement inséré dans l'aiguille, les extrémités distales de l'aiguille et de l'insert étant alignées.

Suivant la position de l'extrémité distale de l'insert par rapport à celle de l'extrémité distale de l'aiguille, il est possible d'obtenir encore d'autres rayons de courbure de l'aiguille et de l'insert.

On comprend donc qu'en choisissant différents comportements pour des régions différentes de l'aiguille et de l'insert, et en choisissant la longueur et la position de ces différentes régions, on peut obtenir des aiguilles dont on peut modifier la trajectoire en ajustant la position axiale de l'insert à l'intérieur de l'aiguille.

Enfin, bien que l'alliage nickel-titane se prête particulièrement bien à la mise en oeuvre de l'invention, l'aiguille et l'insert peuvent être en tout matériau biocompatible qui se prête à un traitement thermique ou thermomécanique permettant de modifier de manière localisée les propriétés, notamment le comportement mécanique, de l'aiguille et de l'insert.

De manière générale, on choisit des matériaux élastiques, élastoplastiques ou superélastiques à la température du corps humain.

De préférence, on choisit un matériau présentant une élasticité suffisante pour permettre à l'aiguille d'adopter différentes trajectoires courbes tout étant apte à revenir à sa forme initiale.

### Procédé de fabrication du dispositif

On va maintenant décrire un procédé préféré de fabrication du dispositif qui vient d'être décrit.

On met en oeuvre, sur l'aiguille et/ou sur l'insert, un traitement thermique ou thermomécanique localisé de manière à former des régions présentant un comportement différencié dans l'aiguille et/ou l'insert.

Un tel traitement permet de faire varier localement le comportement mécanique de l'aiguille sans modifier l'épaisseur de l'aiguille, celle-ci restant constante sur toute sa longueur. Il en va de même pour l'insert.

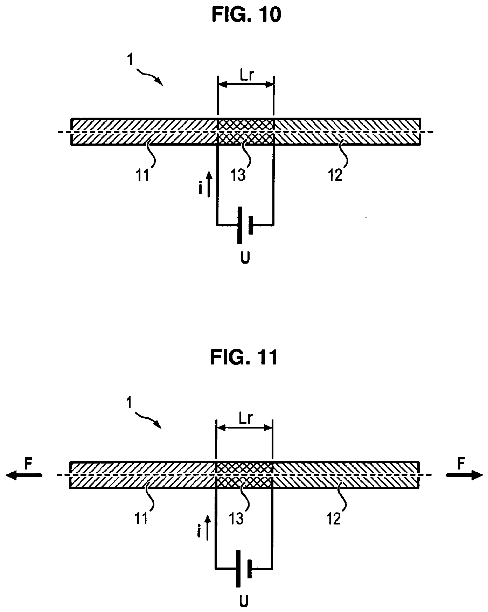

Les figures 10 et 11 illustrent de manière schématique un traitement thermique, respectivement thermomécanique, localisé de l'aiguille 1 par effet Joule.

En référence à la figure 10, on impose une tension U aux bornes d'une portion 13 de l'aiguille 1.

L'aiguille étant en un matériau électriquement conducteur, la portion 13 est parcourue par un courant i et s'échauffe par effet Joule, ce qui modifie ses propriétés élastiques.

En référence à la figure 11 le traitement de la figure 6 est complété par l'application d'une contrainte de traction schématisée par les flèches F sur les extrémités de l'aiguille.

L'application de cette contrainte combinée au traitement thermique permet de modifier les propriétés de la portion 13 sur toute sa circonférence.

Le comportement élastique de la portion 13 ainsi traitée dépend de la durée du traitement, de la tension appliquée, de la longueur Lr de la zone traitée, ainsi que de la contrainte mécanique éventuellement appliquée.

La portion courbée 21 de l'insert peut être obtenue par un procédé dit de « shape setting », c'est-à-dire en imposant la forme souhaitée au matériau, puis en réalisant un traitement thermique adapté.

Pour la mise en oeuvre du traitement thermomécanique, on pourra se référer à l'article de D. Favier et al., "Influence of Thermomechanical processing on the superelastic properties of a Ni-rich Nitinol shape memory alloy", Materials Sciences and Engineering, A429 (2006), qui décrit un traitement thermomécanique de l'ensemble d'une pièce.

Naturellement, tout autre procédé permettant un traitement thermique ou thermomécanique localisé de l'aiguille et/ou de l'insert peut être employé.

On peut citer par exemple un traitement laser, ou un chauffage par induction.

Un traitement laser est en particulier avantageux pour modifier le comportement mécanique de l'aiguille sur une partie seulement de la circonférence de sa portion distale.

Ce traitement permet en effet de modifier de manière contrôlée le comportement mécanique d'une région s'étendant sur un secteur angulaire déterminé de la circonférence de l'aiguille, sans modifier le comportement mécanique d'une région s'étendant sur un autre secteur angulaire de la circonférence, le comportement de cette région pouvant alors être sensiblement identique à celui du reste de l'aiguille.

Ce traitement laser localisé permet en particulier de réaliser l'aiguille illustrée aux figures 6A à 6C.

Dans le cas d'un traitement thermomécanique, la contrainte appliquée à l'aiguille ou à l'insert peut être une contrainte de traction ou une contrainte radiale...

De préférence, le traitement thermique et/ou thermomécanique est réalisé dans une atmosphère neutre, afin d'éviter toute oxydation des matériaux de l'aiguille et de l'insert.

### Utilisation du dispositif de guidage

Lors de l'utilisation du dispositif de guidage de l'aiguille, l'insert est inséré dans l'aiguille de sorte à procurer la courbure souhaitée à la portion distale de l'aiguille.

Au cours de l'insertion de l'aiguille à travers les tissus, le praticien actionne l'insert en translation et/ou en rotation dans l'aiguille de manière à adapter la courbure de l'extrémité distale de l'aiguille en fonction des éventuels obstacles rencontrés et de sa position par rapport à la cible.

De manière particulièrement avantageuse, l'aiguille peut être munie de capteurs qui permettent de déterminer en temps réel sa courbure.

### REFERENCES

Webster et al., "Nonhlonmic modeling of needle steering", International Journal of Robotics Research, Sage Publications Ltd, 2006, 25, pp. 509-525
WO 2010/020591
US 2009/0270676
T.R. Wedlick et al., "Characterization of Pre-Curved Needles for Steering in Tissue", 31st Annual International Conférence of the IEEE EMBS Minneapolis, 26 Sept. 2009
P. Sears et al., "A steerable Needle Technology Using Curved Concentric Tubes", Proceedings of the 2006 IEEE/RSJ International Conférence on Intelligent Robots and Systems, Oct. 9-15, 2006, pp. 2850-2856
D. Favier et al., "Influence of Thermomechanical processing on the superelastic properties of a Ni-rich Nitinol shape memory alloy", Materials Sciences and Engineering, A429 (2006)

## Revendications

1. Dispositif de guidage d'une aiguille médicale, comprenant ladite aiguille médicale (1) et un insert (2) agencé en coulissement en translation et rotation dans l'aiguille (1), l'insert (2) présentant, à l'état libre, une portion distale (21) courbée et l'aiguille présentant au moins deux régions (11, 12) adjacentes dont les comportements mécaniques sont différents, le comportement mécanique d'au moins une région (12) étant obtenu par un traitement thermique ou thermomécanique de l'aiguille, les comportements mécaniques de chacune desdites régions (11, 12) et de la portion distale courbée (21) de l'insert étant choisis de sorte qu'un déplacement de l'insert (2) dans l'aiguille (1) impose une déformation localisée de l'aiguille et/ou de l'insert, ledit dispositif étant **caractérisé en ce que** l'aiguille (1) et l'insert (2) sont en des métaux biocompatibles élastiques, élastoplastiques ou super-élastiques à la température du corps humain, et **en ce que** lesdites au moins deux régions (11, 12) sont adjacentes sur la circonférence de la portion distale de l'aiguille (1).

2. Dispositif selon la revendication 1, **caractérisé en ce que** l'aiguille (1) présente aux moins deux régions (11, 12) de rigidités différentes, l'une au moins desdites régions (11, 12) présentant une rigidité supérieure à celle de la portion courbée (21) de l'insert.

3. Dispositif selon l'une des revendications 1 à 2, **caractérisé en ce que** l'insert présente une forme cylindrique pleine de section circulaire.

4. Dispositif selon l'une des revendications 1 à 3, **caractérisé en ce que** l'aiguille (1) et/ou l'insert (2) sont dans un alliage nickel-titane.

5. Dispositif selon l'une des revendications 1 à 4, **caractérisé en ce que** l'aiguille (1) et l'insert (2) présentent une épaisseur constante.

6. Dispositif selon l'une des revendications 1 à 5, **caractérisé en ce que** le diamètre extérieur de l'aiguille (1) est compris entre 0,3 et 2 mm et le diamètre de l'insert (2) est compris entre 0,1 et 1,8 mm.

7. Dispositif selon l'une des revendications 1 à 6, **caractérisé en ce que** l'aiguille (1) présente, de son extrémité distale vers son extrémité proximale, une première portion (12) dont la rigidité est supérieure à la rigidité de la portion courbée (21, 23) de l'insert (2) et une deuxième portion (13) dont la rigidité est inférieure à la rigidité de la portion courbée (21, 23) de l'insert (2).

8. Procédé de fabrication d'un dispositif de guidage d'une aiguille médicale, ledit dispositif comprenant ladite aiguille médicale (1) et un insert (2) agencé en coulissement en translation et rotation dans l'aiguille (1), procédé selon lequel on applique à l'aiguille et à l'insert un traitement thermique et/ou thermomécanique localisé pour former :
- sur l'insert (2), au moins une portion distale courbée (21) à l'état libre,
- sur l'aiguille (1), au moins deux régions présentant des comportements mécaniques différents, les comportements mécaniques de chacune desdites régions (11, 12) de l'aiguille et de la portion distale courbée (21) de l'insert étant choisis de sorte que qu'un déplacement de l'insert (2) dans l'aiguille (1) impose une déformation localisée de l'aiguille et/ou de l'insert, ledit procédé étant **caractérisé en ce que** l'aiguille (1) et l'insert (2) sont en des métaux biocompatibles élastiques, élastoplastiques ou super-élastiques à la température du corps humain, et **en ce que** lesdites au moins deux régions (11, 12) sont adjacentes sur la circonférence de la portion distale de l'aiguille (1).

9. Procédé selon la revendication 8, **caractérisé en ce que** le traitement thermique et/ou thermomécanique appliqué à l'aiguille (1) forme au moins deux régions de rigidités différentes, l'une au moins desdites portions présentant une rigidité supérieure à la rigidité de la portion courbée (21) de l'insert.

10. Procédé selon l'une des revendications 8 ou 9, **caractérisé en ce que** ledit traitement thermique ou thermomécanique est réalisé dans une atmosphère neutre.

11. Procédé selon l'une des revendications 8 à 10, **caractérisé en ce que** ledit traitement thermique ou thermomécanique comprend une irradiation localisée de l'aiguille par un laser.

## Patentansprüche

1. Vorrichtung zum Führen einer medizinischen Kanüle, umfassend die medizinische Kanüle (1) und einen Einsatz (2), der verschiebend und rotierend gleitend in der Kanüle (1) ausgebildet ist, wobei der Einsatz (2) in freiem Zustand einen gekrümmten distalen Abschnitt (21) aufweist und die Kanüle mindestens zwei benachbarte Regionen (11, 12) aufweist, deren mechanische Verhaltensweisen unterschiedlich sind, wobei die mechanische Verhaltensweise mindestens einer Region (12) durch eine thermische oder thermomechanische Behandlung der Kanüle erhalten wird, wobei die mechanischen Verhaltensweisen jeder der Regionen (11, 12) und des gekrümmten distalen Abschnitts (21) des Einsatzes derart ausgewählt sind, dass eine Verlagerung des Einsatzes (2) in der Kanüle (1) eine lokalisierte Verformung der Kanüle und/oder des Einsatzes bewirkt, wobei die Vorrichtung **dadurch gekennzeichnet ist, dass** die Kanüle (1) und der Einsatz (2) aus bei Temperatur des menschlichen Körpers elastischen, elastoplastischen oder superelastischen biologisch verträglichen Materialien sind und dass die mindestens zwei Regionen (11, 12) auf dem Umfang des distalen Abschnitts der Kanüle (1) benachbart sind.

2. Vorrichtung nach Anspruch 1, **dadurch gekennzeichnet, dass** die Kanüle (1) mindestens zwei Regionen (11, 12) unterschiedlicher Steifigkeiten aufweist, wobei mindestens eine der Regionen (11, 12) eine Steifigkeit aufweist, die höher als die des gekrümmten Abschnitts (21) des Einsatzes ist.

3. Vorrichtung nach einem der Ansprüche 1 bis 2, **dadurch gekennzeichnet, dass** der Einsatz eine massive zylindrische Form mit kreisförmigem Querschnitt aufweist.

4. Vorrichtung nach einem der Ansprüche 1 bis 3, **dadurch gekennzeichnet, dass** die Kanüle (1) und/oder der Einsatz (2) aus einer Nickel-Titan-Legierung sind.

5. Vorrichtung nach einem der Ansprüche 1 bis 4, **dadurch gekennzeichnet, dass** die Kanüle (1) und der Einsatz (2) eine konstante Stärke aufweisen.

6. Vorrichtung nach einem der Ansprüche 1 bis 5, **dadurch gekennzeichnet, dass** der Außendurchmesser der Kanüle (1) zwischen 0,3 und 2 mm beträgt und der Durchmesser des Einsatzes (2) zwischen 0,1 und 1,8 mm beträgt.

7. Vorrichtung nach einem der Ansprüche 1 bis 6, **dadurch gekennzeichnet, dass** die Kanüle (1) von ihrem distalen Ende zu ihrem proximalen Ende einen ersten Abschnitt (12) aufweist, dessen Steifigkeit höher als die Steifigkeit des gekrümmten Abschnitts (21, 23) des Einsatzes (2) ist und einen zweiten Abschnitt (13), dessen Steifigkeit niedriger als die Steifigkeit des gekrümmten Abschnitts (21, 23) des Einsatzes (2) ist.

8. Verfahren zur Herstellung einer Führungsvorrichtung einer medizinischen Kanüle, wobei die Vorrichtung die medizinische Kanüle (1) und einen Einsatz (2) umfasst, der verschiebend und rotierend gleitend in der Kanüle (1) ausgebildet ist, wobei bei dem Verfahren die Kanüle und der Einsatz einer lokalisierten thermischen und/oder thermomechanischen Behandlung unterzogen werden, um zu bilden:
- auf dem Einsatz (2), mindestens einen in freiem Zustand gekrümmten distalen Abschnitt (21),
- auf der Kanüle (1), mindestens zwei Regionen, die unterschiedliche mechanische Verhaltensweisen aufweisen,
wobei die mechanischen Verhaltensweisen jeder der Regionen (11, 12) der Kanüle und des gekrümmten distalen Abschnitts (21) des Einsatzes derart ausgewählt sind, dass eine Verlagerung des Einsatzes (2) in der Kanüle (1) eine lokalisierte Verformung der Kanüle und/oder des Einsatzes bewirkt, wobei das Verfahren **dadurch gekennzeichnet ist, dass** die Kanüle (1) und der Einsatz (2) aus bei Temperatur des menschlichen Körpers elastischen, elastoplastischen oder superelastischen biologisch verträglichen Materialien sind und dass die mindestens zwei Regionen (11, 12) auf dem Umfang des distalen Abschnitts der Kanüle (1) benachbart sind.

9. Verfahren nach Anspruch 8, **dadurch gekennzeichnet, dass** die auf die Kanüle (1) angewendete thermische und/oder thermomechanische Bearbeitung mindestens zwei Regionen (11, 12) unterschiedlicher Steifigkeiten bildet, wobei mindestens einer der Abschnitte eine Steifigkeit aufweist, die höher als die Steifigkeit des gekrümmten Abschnitts (21) des Einsatzes ist.

10. Verfahren nach einem der Ansprüche 8 oder 9, **dadurch gekennzeichnet, dass** die thermische oder thermomechanische Behandlung in einer neutralen Atmosphäre durchgeführt wird.

11. Verfahren nach einem der Ansprüche 8 bis 10, **dadurch gekennzeichnet, dass** die thermische oder thermomechanische Behandlung eine lokalisierte Bestrahlung der Kanüle durch einen Laser umfasst.

## Claims

1. A device for guiding a medical needle, comprising said medical needle (1) and an insert (2) arranged slidably in translation and rotation inside the needle (1), the insert (2), in a free condition, having a curved distal portion (21) and the needle having at least two adjacent regions (11, 12) having different mechanical behaviours, the mechanical behaviour of at least a region (12) being obtained by heat or thermo-mechanical treatment of the needle, the mechanical behaviours of each of said regions (11, 12) and of the curved distal portion (21) of the insert being chosen so that movement of the insert (2) inside the needle (1) causes localised deformation of the needle and/or the insert, said device being **characterized in that** the needle (1) and the insert (2) are in biocompatible metals that are elastic, elasto-plastic or super-elastic at the temperature of the human body, and **in that** said at least two regions (11, 12) are adjacent over the circumference of the distal portion of the needle (1).

2. The device according to claim 1, **characterized in that** the needle (1) has at least two regions (11, 12) of different rigidity, at least one of said regions (11, 12) having greater rigidity than the curved portion (21) of the insert.

3. The device according to one of claims 1 to 2, **characterized in that** the insert has a solid cylindrical shape of circular cross-section.

4. The device according to one of claims 1 to 3, **characterized in that** the needle (1) and/or the insert (2) are in nickel-titanium alloy.

5. The device according to one of claims 1 to 4, **characterized in that** the needle (1) and the insert (2) are of constant thickness.

6. The device according to one of claims 1 to 5, **characterized in that** the outer diameter of the needle (1) is between 0.3 and 2 mm and the diameter of the insert (2) is between 0.1 and 1.8 mm.

7. The device according to one of claims 1 to 6 **characterized in that** the needle (1) has, from its distal tip to its proximal end, a first portion (12) having greater rigidity than the rigidity of the curved portion (21, 23) of the insert (2), and a second portion (13) having smaller rigidity than the curved portion (21, 23) of the insert (2).

8. A method for manufacturing a device for guiding a medical needle, said device comprising said medical needle (1) and an insert (2) arranged slidably in translation and rotation inside the needle (1), the method according to which the needle and the insert are subjected to localised heat and/or thermo-mechanical treatment to form:
- on the insert (2), at least one curved distal portion (21) in a free condition,
- on the needle (1), at least two regions having different mechanical behaviours,
the mechanical behaviours of each of said regions (11, 12) of the needle and of the curved distal portion (21) of the insert being chosen so that movement of the insert (2) in the needle (1) causes localised deformation of the needle and/or insert, said method being **characterized in that** the needle (1) and the insert (2) are in biocompatible metals that are elastic, elasto-plastic or super-elastic at the temperature of the human body, and **in that** said at least two regions (11, 12) are adjacent over the circumference of the distal portion of the needle (1).

9. The method according to claim 8, **characterized in that** the heat and/or thermo-mechanical treatment applied to the needle (1) forms at least two regions of different rigidity, at least one of said portions having greater rigidity than the rigidity of the curved portion (21) of the insert.

10. The method according to one of claims 8 or 9, **characterized in that** said heat or thermo-mechanical treatment is conducted in a neutral atmosphere.

11. The method according to one of claims 8 to 10, **characterized in that** said heat or thermo-mechanical treatment comprises localised laser irradiation of the needle.
